# EUROPEAN PATENT APPLICATION

(11) **EP 2 080 509 A1**
(43) Date of publication of application: **22.07.2009**
(21) Application number: 09250105.5
(22) Date of filing: 16.01.2009
(51) Int. Cl.: A61K 9/00, A61K 47/10, A61K 47/34

(54) **Lubricious, non-tacky personal lubricant**

(30) Priority: 18.01.2008 US 16651
(71) Applicant: McNeil-PPC, Inc., Skillman, NJ 08558 (US)
(72) Inventor: Vochecowicz, Stacy, Hillsborough, NJ 08844 (US); Fevola, Michael J., Belle Mead, NJ 08502 (US)
(74) Representative: Kirsch, Susan Edith

(57) **Abstract**

This invention relates to personal lubricant compositions that are relatively non-tacky while maintaining lubricity.

## Description

### Field of the Invention

This invention relates to a composition useful for personal lubrication during intimate contact that is highly lubricious and non-tacky.

### Background of the Invention

Personal lubricants for intimate contact are well known. Typically, personal lubricants are marketed as liquids, jellies, gels or suppositories. Examples of such products include K-Y® Jelly, Astroglide®, K-Y® Liquid, K-Y® Ultragel^{™}. More recently K-Y® Warming Liquid was introduced to the marketplace. K-Y ® Warming Liquid is a water soluble, anhydrous composition that warms on contact while providing lubrication.

Personal lubricants must be lubricious, however, many known personal lubricants that are in the form of jellies or gels have higher viscosities but can be perceived as "tacky" or "sticky" by the users.

Therefore, there exists a need for a non-tacky, lubricious composition for personal lubrication use that has appropriate viscosity for intimate use.

### SUMMARY OF THE INVENTION

We have discovered that, unexpectedly, imparting certain physical characteristics to personal lubricant compositions endows them with the capability of being lubricious yet non-tacky and sufficiently viscous to be effective as a personal lubricant.

The present invention provides a composition for personal lubrication comprising at least one polyhydric alcohol and a hydrophobically-modified polymer, wherein said composition has a tackiness of no less than about -27 and a lubricity of at least about 8.

The present invention also discloses a method of providing personal lubrication to the skin of an individual comprising applying to said individual's skin a composition as described herein.

The present invention also discloses the use of a composition described herein for providing personal lubrication to the skin of an individual.

Preferably, the personal lubricant compositions of this invention contain at least one polymeric thickener, at least one polyhydric alcohol and water. Preferably, but optionally, the personal lubricant compositions of this invention should further contain a preservative system. The personal lubricant compositions of this invention should have a lubricity of at least about 8, more preferably at least about 15, more preferably at least about 20 and most preferably, at least about 30.

Preferably, the personal lubricant compositions of this invention should have a viscosity of from about 50,000 to about 200,000 centipoise ("cps"). They should have a pH that is compatible with the vagina. Preferably, the pH should be between about 3.5 and about 5.5. They should be visually clear and translucent to the eye, non-hazy and not containing any undissolved particles. The composition may contain air bubbles. The composition should be non-sticky, having a tackiness of no less than about -27 g for the first cycle and at least no less than -25 g for the second cycle. More preferably, at least no less than -26.5 g for the first cycle and at least no less than -24 g for the second cycle. More preferably, at least no less than -22 g for the first cycle and at least no less than -20 g for the second cycle. The composition is preferably odorless and easily dispensible out of a packaging component.

The personal lubricant compositions of this invention should contain from about 0.1 to about 3% by weight of a hydrophobically-modified polymer and from about 0 to about 50% by weight of polyhydric alcohols.

### DETAILED DESCRIPTION OF THE INVENTION

In one embodiment, the compositions of this invention contain at least one polyhydric alcohol that is water-soluble, and a hydrophobically-modified polymer.

In one embodiment, the composition of the present invention contains at least one polyhydric alcohol. The at least one polyhydric alcohol fraction of this composition may contain propylene glycol, polyethylene glycol, glycerol, sorbitol or a combination thereof. Polyethylene glycol may be selected ranging in molecular weight of from 300 to about 1450. The polyhydric alcohol portion of the compositions of this invention should make up from about 5 to about 50% by weight of the composition. More preferably the compositions of this invention should contain a combination of two or more polyhydric alcohols. Most preferably, the polyhydric portion of the composition should contain glycerin and sorbitol. Preferably there should be from about 5 to about 50% by weight of sorbitol, more preferably from about 5 to about 30% by weight of sorbitol, more preferably from about 5 to about 20% by weight sorbitol and/or preferably from about 2 to about 40% by weight of glycerin, preferably from about 5% to about 30% by weight glycerin, preferably from about 5% to about 20% by weight glycerin. The ratio of glycerin to sorbitol should be from about 40:60 to about 60:40, more preferably about 60:40.

Examples of the hydrophobically-modified polymer include polyacrylics and acrylate crosspolymers. Hydrophobically modified polymers useful in the compositions of this invention include the following: Acritamer 501ED, Acritamer 505ED, Aqupec HV-501ER, Carbopol ETD 2020, Carbopol 1342, Carbopol 1382, Carbopol Ultrez 20, Carbopol Ultrez 21, Pemulen TR-1, Pemulen TR-2 and Tego Carbomer 341 ER, Aculyn 88, Aculyn 22, Structure 2001, Balance Gel, Structure 3001. (Corresponding INCI names are Acrylates/C10-C30 Alkyl Acrylate Crosspolymer, Acrylates/Steareth-20 Methacrylate Crosspolymer, Acrylates/Steareth-20 Methacrylate Copolymer, Acrylates/Steareth-20 Itaconate Copolymer, Acrylates/Ceteth-20 Itaconate Copolymer). Such hydrophobically-modified polymers are also set forth in U.S. Patent No. 6,433,061 (Marchant et.al) which is hereby incorporated by reference.

A preservative may be important for use in the products of this invention, in order to preserve the stability of the compositions of this invention and to prevent the growth of microorganisms therein. The compositions of the present invention may contain one or more preservatives selected from the group consisting of methylparaben, phenoxyethanol, benzoic acid, sorbic acid, gallic acid, propylparaben, sodium benzoate, potassium sorbate and combinations thereof. Alternatively, the preservative portion of the compositions of this invention may be one or more known preservatives, such as methylparaben, phenoxyethanol, benzoic acid, sorbic acid, gallic acid or propylparaben. The preservative may be present in an amount of from about 0.05% to about 1% by weight of the composition, preferably about 0.1% to about 0.9% by weight of the composition, preferably about 0.3% to about 0.9% by weight of the composition, preferably about 0.6% to about 0.9% by weight of the composition, preferably about 0.8% by weight of the composition.

The compositions of the present invention may include organic acids selected from the group consisting of benzoic acid, citric acid, linolic acid, oxalic acid, ketoglutaric acid, tannic acid, humic acid, glycolic acid, gallic acid, malic acid and combinations thereof. Alternatively, the compositions may include organic acids such as benzoic acid, citric acid, linolic acid, oxalic acid, ketoglutaric acid, tannic acid, humic acid, glycolic acid, gallic acid and malic acid. The pH of the composition should be adjusted to be between about 3.5 and about 5.5 in order to maintain compatibility with the vaginal pH. pH can be adjusted with citric acid, sodium hydroxide, triethanolamine, lactic acid, potassium hydroxide or the like.

However, it should be noted that pH affects the viscosity of the compositions of this invention in that lower pH decreases the viscosity of the hydrophobically-modified polymers useful in the compositions of this invention. Likewise, lowering the concentration of hydrophobically-modified polymer in the compositions of this invention lowers their viscosity as well. Thus, a balance needs to be struck between the desired and appropriate viscosity of the compositions of this invention, their pH and the concentration of hydrophobically-modified polymer in the compositions.

Water, which makes up the remaining portion of the compositions of this invention functions to provide appropriate consistency, viscosity and lubricity of the compositions.

Other ingredients which may be included in the compositions of this invention preferably include antioxidants, chelators, preservatives and pH adjustors.

The compositions of the present invention may comprise an antioxidant such as ascorbyl palmitate, benzoic acid, butylated hydroxytolouene, butylated hydroxyanisole, ethylenediaminetetraacetic acid and its sodium salts, potassium sorbate, sodium benzoate, propylparaben, sodium bisulfite, sassafras oil, sodium metbisulfite, sorbic acid, maleic acid and propyl gallate and the like. The antioxidant may be present in an amount of from about 0.001% to about 1% by weight of the composition.

The composition of the present invention may include a pH adjustor. The pH adjuster may be selected from the group consisting of sodium hydroxide, citric acid, triethanolamine, lactic acid and potassium hydroxide. Alternatively, the pH adjustors may include sodium hydroxide, citric acid, triethanolamine and potassium hydroxide and the like. Mixtures of the aforementioned pH adjustors may also be used. Preferably the pH adjuster is present in an amount of from about 0.001% to about 1% of the composition, more preferably from about 0.01% to 0.5% by weight of the composition, more preferably from about 0.01% to about 0.05% by weight of the composition.

We have found that the compositions of this invention provide unexpectedly high lubricity and relatively low tackiness at similar viscosity to personal lubricant compositions that contain water-soluble cellulose-derived polymers.

Yet other embodiments of the compositions of this invention are compositions that may include local anesthetics. The local anesthetic may be selected from the group consisting of benzocaine, lidocaine, dibucaine, benzyl alcohol, camphor, resorcinol, menthol, diphenylhydramine hydrochloride or mixtures thereof. Alternatively, the local anesthetics may preferably include, but are not limited to, benzocaine, lidocaine, dibucaine, benzyl alcohol, camphor, resorcinol, menthol and diphenylhydramine hydrochloride and the like.

Compositions of the invention may also include plant extracts such as aloe, witch hazel, chamomile, hydrogenated soy oil and colloidal oatmeal, vitamins such as vitamin A, D or E and corticosteroids such as hydrocortisone acetate.

### Lubricity

The compositions of the present invention provide personal lubrication. Personal lubricants prevent irritation, which may result due to friction. As an example, some post-menopausal women find sexual intercourse painful due to dryness of the vagina. The use of a personal lubricant helps to overcome this condition.

Lubricity may be measured using the following test method known herein as the ***"Ahmad Procedure**"* which was described in U.S Patent No. 6,139,848. Briefly, the test method measures the amount of force required to move one surface relative to another while under weight pressure (the two surfaces being horizontal to each other). A weight or pressure can be applied on the upper moving surface. The test composition, in this case, the test lubricants, work by reducing the friction between the two surfaces. From this force and weight, a coefficient of friction value for a lubricant can be calculated. The coefficient of friction is inversely proportional to the lubricity of a product and is known as "relative lubricity". Relative lubricity can be calculated from the coefficient of friction data by dividing the numeral one by the corresponding coefficient of friction value.

An instrument, namely Coefficient of Sliding Friction Rig adapted to a Texture Analyzer, marketed by Texture Technologies Corp., 18 Fairview Road, Scarsdale, N.Y. was used for determining relative lubricity of several lubricant products in comparison with that of the compositions of this invention. The equipment contains a platform having a friction sledge attached to a load cell which is constrained to slide across the platform over which a test sample is applied. Load is provided by a 430 g precision weight positioned centrally over the sledge. This arrangement offers the advantage of measuring coefficient of sliding friction in both directions such that data for the "push" and the "pull" phases of the test can be generated over a fixed period of time. For the examples set forth below, it was possible to generate coefficient of friction data for an extended period of five (5) minutes. In making the measurements for the examples, a non-lubricated condom was mounted over the sledge, a thin film of the lubricant sample was applied over the fixed platform and coefficient of sliding friction readings were recorded over a five-minute period while the friction sledge went back and forth from the starting point. The coefficient of friction data, therefore, has negative (-) sign during the "push" phase and positive (+) sign during the "pull" phase of the experiment. The coefficient of friction data for the baseline, with no product applied to the condom was also generated for comparison. Texture Analyzer TA-XT2I (SID 41) was utilized for the test, having a Plexiglas^{™} plate 3" x 4" x 3/8" in size, a 430 g weight and a 6.0 mil Bird Applicator. The substrate used was a polyethylene/foil liner and a Trojans^{®} non-lubricated condom. The texture analyzer settings were as follows: Test Mode and Option, Measure Force In tension, Cycle Until Count, Trigger, Type-Button, Stop Plot at--Trigger Return, Brea--Detect off, level. The pre-test speed was 0 mm/s, the test speed was 2.0 mm/s, the post test speed was 0.0 mm/s and the distance traveled was 40.0 mm. The test was run for 300 seconds. The PE/foil liner was glued to the aluminum base or platform of the Texture Analyzer. The Plexi-glas^{™} sled was covered with the condom, a 6.0 mil film of test product was cast onto the liner and the 430 g weight placed on the center of the sled.

Preferably, the lubricity range for the compositions of this invention as determined using the Ahamd procedure should be at least about 8 for the duration of between about 100 and about 900 seconds, more preferably, more than about 30 for the duration of between about 100 and about 900 seconds.

### Viscosity

In order for the composition to be useful as a personal lubricant and aesthetically pleasing to the user, the compositions of this invention preferably have a viscosity that allows the compositions to be applied easily to the body and spread over the skin in an even manner. It has been found that while achieving the appropriate viscosity, maintaining lubricity is equally important.

### Tackiness

The tackiness (or "stickiness") of each of the prototypes was determined by the Tack Method described in detail below. The results indicate that prototypes containing the HM polymer display lower tack values than the prototypes containing a cellulosic polymer, while keeping viscosity of the prototypes constant.

The Tack Method measures the amount of force required to pull one surface away from a treated surface. The prototypes are evaluated on tack measured between the treated substrate and the instrument probe. From the negative force that is observed while the probe ascends, and pulls away from the treated substrate, we can obtain the maximum (negative) force.

An instrument, namely a Texture Analyser, marketed by Texture Technologies Corp., 18 Fairview Road, Scarsdale, N.Y. was used for determining tackiness of the prototypes. Texture Analyzer TA-XT Plus was utilized for the test. The equipment contains a round, 2.5 mm diameter, acrylic probe and an aluminum base or platform on which a rubber substrate is positioned. In making the measurements for the prototypes, 15 microliters of the product are applied, using a positive displacement pipette, to a circular area on the center of the substrate, measuring 4.91 cm². The product is spread over the circular area using the tip of the pipette. Within 45 seconds of initially depositing the product on the substrate, the test is initiated and the probe descends onto the treated substrate. The probe will descend onto the treated substrate until a force of 250 grams is observed. At that point, the probe will ascend, and the force therefore has a negative (-) sign during the ascension phase, which allows measurement of the maximum negative force associated with pulling the probe and treated substrate apart, hence measuring the tack of the product (or treatment). This test can be generated over a fixed period of cycles, in which one cycle constitutes the probe descending onto the substrate and ascending back to a fixed point. Preferably, the tack data is generated for two cycles. Two cycles is chosen to avoid drying of the product, due to evaporation. Drying would create an artifact at longer cycle times.

The texture analyzer settings should be as follows: Test Mode is compression, Measure Force In grams, Repeat Until Count, Trigger Type-Force. The pre-test speed should be 2 mm/s, the test speed should be 2 mm/s, the post test speed should be 2 mm/s and the distance traveled should be 1.25mm after trigger. The test is run for two cycles. The rubber substrate is positioned on the aluminum base or platform of the Texture Analyzer, directly under the probe. The rubber substrate is held down with weights, totaling 925 g to ensure immobility.

Preferably, the tack range for the compositions of this invention as determined using the above procedure should be at least no less than -27 g for the first cycle and at least no less than -25 g for the second cycle. More preferably, at least no less than - 26.5 g for the first cycle and at least no less than -24 g for the second cycle.

### Rheology

The rheology of each of the prototypes was determined by the Frequency Sweep Method described in detail below. The results indicate that over the range described below, prototypes containing the hydrophobically modified polymer display purely elastic gel properties, whereas the prototypes containing a cellulosic polymer displays viscoelastic fluid properties. Elastic defines materials that, when strained, will recover completely. Viscoelastic materials are those that, when strained, will recover partially but there is some element of permanent deformation.

A controlled stress rheometer, marketed by TA Instruments, 109 Lukens Drive, New Castle, DE was used for measuring rheology of the prototypes. TA Instrument AR-G2 was utilized for the test and was equipped with a 20mm 2" steel cone and Peltier plate.

The basic Frequency Sweep settings for the TA Instrument were set as follows: Angular Frequency (rad/s) range was set as 0.1 to 100.0; G' and G" measured in log mode; % strain kept constant.

The following examples serve to illustrate the compositions and methods of this invention. However, they are not presented in order to limit the scope of the invention in any way. Examples 1, 2 and 3 were prepared as follows containing quantities listed in the tables below. Deionized water was added to a mixing vessel, while agitation and heating (to around 52°C) were initiated. A hydrophobically modified polymer was added and mixed until fully wetted out. Once the temperature of the mixture reached 50°C, the methylparaben was added. Mixing continued until the mixture was uniform. Once uniformity was achieved, the glycerin was added. Heat was then turned off and once the mixture reached 45-50°C, the sorbitol and phenoxyethanol were added, while mixing continued. Once the temperature of the mixture reached 30°C or below, the pH was adjusted with sodium hydroxide. Mixing speed was adjusted during the process to decrease aeration and facilitate the homogeneity of the composition.

Example 4 was prepared as follows containing quantities as listed according to the table below. Deionized water was added to a mixing vessel, while agitation and heating to 60°C were initiated. A cellulosic polymer was added and mixed until dissolved. Once the mixture was dissolved, the methylparaben was added. Mixing continued until uniform and clear. Once uniformity and clarity was achieved, heat was turned off and the glycerin, sorbitol and phenoxyethanol were added. Mixing speed was adjusted during the process to decrease aeration and facilitate the homogeneity of the composition.

Example 5 was prepared as follows containing quantities as listed according to the table below. Deionized water was added to a mixing vessel, while agitation and heating to 45°C were initiated. The methylparaben was added and mixed until dissolved. Once uniformity and clarity was achieved, glucono delta lactone was added. With continued mixing, sodium hydroxide and then chlorhexidine gluconate were added. In a side mixing vessel, the glycerin and cellulosic polymer were combined and agitation initiated. Once the glycerin/polymer side phase was homogeneous and free of undispersed material, the side phase was added to the main water phase. Mixing continued until uniform. Mixing speed was adjusted during the process to decrease aeration and facilitate the homogeneity of the composition

The methods of the present invention may further comprise any of a variety of steps for mixing or introducing one or more of the optional components described hereinabove with or into a composition comprising a hyrophobically modified polymer either before, after, or simultaneously with the combining step described above. While in certain embodiments, the order of mixing is not critical, it is preferable, in other embodiments, to pre-blend certain components, such as the fragrance and the nonionic surfactant before adding such components into a composition comprising the polymerized surfactant

### Example 1 - Lubricant containing hydrophobically modified polymer

| **Ingredient** | **%w/w** |
|---|---|
| Purified Water, USP | 81.445 |
| Glycerin, USP | 10.000 |
| Sorbitol | 7.000 |
| Acrylates/C10-30 Alkyl | |
| Acrylate Crosspolymer | 0.725 |
| Phenoxyethanol | 0.600 |
| Methylparaben | 0.200 |
| Sodium Hydroxide | 0.030 |
| Total | 100.000 |

### Example 2 - Lubricant containing hydrophobically modified polymer

| **Ingredient** | **%w/w** |
|---|---|
| Purified Water, USP | 87.360 |
| Glycerin, USP | 6.500 |
| Sorbitol | 4.500 |
| Acrylates/Steareth-20 | |
| Methacrylate Crosspolymer | 0.900 |
| Phenoxyethanol | 0.600 |
| Methylparaben | 0.200 |
| Sodium Hydroxide | 0.040 |
| Total | 100.000 |

### Example 3 - Lubricant containing hydrophobically modified polymer

| **Ingredient** | **%w/w** |
|---|---|
| Purified Water, USP | 87.360 |
| Glycerin, USP | 12.000 |
| Sorbitol | 5.000 |
| Acrylates/Ceteth₋20 | |
| Itaconate Copolymer | 1.000 |
| Phenoxyethanol | 0.600 |
| Methylparaben | 0.200 |
| Sodium Hydroxide | 0.010 |
| Total | 100.000 |

### Example 4 (Comparative Example) - Lubricant containing cellulosic polymer

| **Ingredient** | **%w/w** |
|---|---|
| Purified Water, USP | 86.580 |
| Glycerin, USP | 6.500 |
| Sorbitol | 4.500 |
| Hydroxyethylcellulose, NF | 1.600 |
| Phenoxyethanol | 0.600 |
| Methylparaben | 0.200 |
| Citric Acid | 0.020 |
| Total | 100.000 |

### Example 5 (Comparative Example) - Lubricant containing cellulosic polymer

| **Ingredient** | **%w/w** |
|---|---|
| Purified Water, USP | 79.660 |
| Glycerin, USP | 17.000 |
| Hydroxyethylcellulose, NF | 2.300 |
| Chlorhexidine Gluconate | 0.250 |
| Methylparaben | 0.200 |
| Gluconolactone | 0.500 |
| Sodium Hydroxide | 0.090 |
| Total | 100.000 |

### Example 6: Lubricity

The compositions set forth in Examples 1, 4 and 5 were tested using the Ahmad Procedure described above and in U.S. Patent No. 6,139,848. As shown in Table 1 below, the lubricity of the compositions of this invention is significantly and unexpectedly higher than that of lubricant compositions containing cellulosic polymers having similar viscosities. The viscosities of Examples 1, 4 and 5 were 66,000 cps, 65,000 cps and 72,000 cps respectively.

**Table 1**

| Example 1 - Lubricant w/ Hydrophobically modified polymer | | Example 4 - Lubricant w/Cellulosic Polymer | | Example 5 - Lubricant w/Cellulosic Polymer | |
|---|---|---|---|---|---|
| Time | Calculated Lubricity | Time | Calculated Lubricity | Time | Calculated Lubricity |
| (s) | (1/ Coef) | (s) | (1/ Coef) | (s) | (1/ Coef) |
| 96 | 32.6 | 96 | 5.3 | 96 | 4.2 |
| 104 | 32.6 | 104 | 4.9 | 104 | 4.1 |
| 896 | 9.2 | 896 | 4.1 | 896 | 3.2 |
| 904 | 9.3 | 904 | 3.8 | 904 | 3.1 |

### Example 7: Tackiness

The compositions set forth in Examples 1, 4 and 5 were tested using the Tack Method described above. As shown in Table 2 below, the tackiness of the compositions of this invention is significantly and unexpectedly lower than that of lubricant compositions containing cellulosic polymers having similar viscosities. The viscosities of Examples 1, 4 and 5 were 66,000 cps, 65,000 cps and 72,000 cps respectively.

**Table 2**

| | **RUN 1** | | | **RUN 2** | | | |
|---|---|---|---|---|---|---|---|
| | Force (g) | Distance (mm) | Cycle | Force (g) | Distance (mm) | Cycle | Average Force (g) |
| Example 1 - Lubricant w/Hydrophobica lly Modified Polymer | -21.5 | 0.034 | 1 | -19.7 | 0.054 | 1 | -20.6 |
| | -20.5 | 0.049 | 2 | -19.1 | 0.054 | 2 | -19.8 |
| Example 4 - Lubricant w/Cellulosic Polymer | -29.3 | 0.044 | 1 | -28 | 0.004 | 1 | -28.65 |
| | -27.2 | 0.037 | 2 | -24.5 | 0.003 | 2 | -25.85 |
| Example 5 - Lubricant w/Cellulosic Polymer | -36.4 | 0.052 | 1 | -33.6 | -0.078 | 1 | -35 |
| | -26.5 | 0.052 | 2 | -25.7 | -0.011 | 2 | -26.1 |

### Example 8: Rheology

The compositions set forth in Examples 1 and 4 were tested using the Rheology method described above. As shown in Table 3 below, the composition of this invention can further be distinguished from that of lubricant compositions containing cellulosic polymers. The composition containing the hydrophobically modified polymer creates a purely elastic gel in which the plot of G' and G" do not crossover. The composition containing the cellulosic polymer is a viscoelastic fluid in which the plot of G' and G" crossover at an approximate angular frequency of 1.0 radian/second (rad/s).

**Table 3**

| | Example 1 | | Example 2 | |
|---|---|---|---|---|
| Angular Frequency | G' | G'' | G' | G'' |
| rad/s | Pa | Pa | Pa | Pa |
| 0.1 | 287.8 | 38.48 | 7.447 | 13.84 |
| 0.1585 | 299.7 | 29.78 | 10.99 | 18.3 |
| 0.2512 | 312 | 25.81 | 16.03 | 23.97 |
| 0.3981 | 318.4 | 24.44 | 22.82 | 30.74 |
| 0.631 | 325.2 | 24.27 | 31.93 | 38.47 |
| 1 | 331.6 | 23.89 | 43.5 | 47.07 |
| 1.585 | 337.8 | 25.51 | 57.99 | 56.58 |
| 2.512 | 344.5 | 27.01 | 75.66 | 66.09 |
| 3.981 | 351.9 | 28.24 | 96.54 | 76.02 |
| 6.31 | 359.5 | 32.22 | 120.3 | 85.89 |
| 10 | 369.1 | 36.27 | 147.2 | 95.02 |
| 15.85 | 380.1 | 41.34 | 176.5 | 103.7 |
| 25.12 | 392.6 | 47.36 | 207.5 | 111.7 |
| 39.81 | 408.6 | 54.64 | 239.1 | 118.7 |
| 63.1 | 433.2 | 63.88 | 268.2 | 125.7 |
| 100 | 470.3 | 76.7 | 283.5 | 133.7 |

## Claims

1. A composition for personal lubrication comprising at least one polyhydric alcohol and a hydrophobically-modified polymer, wherein said composition has a tackiness of no less than about -27 g and a lubricity of at least about 8.

2. A composition according to claim 1, wherein said polyhydric alcohol is selected from propylene glycol, polyethylene glycol, glycerine, sorbitol or a combination thereof.

3. A composition according to claim 1 or claim 2, wherein said polyhydric alcohol is present in an amount of less than about 50% by weight of the composition.

4. A composition according to any one of claims 1 to 3, wherein said hydrophobically modified polymer is selected from the group consisting of Acrylates/C10-C30 Alkyl Acrylate Crosspolymer, Acrylates/Steareth-20 Methacrylate Crosspolymer, Acrylates/Steareth-20 Methacrylate Copolymer, Acrylates/Steareth-20 Itaconate Copolymer, Acrylates/Ceteth-20 Itaconate Copolymer.

5. A composition according to any one of claims 1 to 4, wherein said hydrophobically-modified polymer is present in an amount of from about 0.1 to about 3% by weight of the composition.

6. A composition according to any one of claims 1 to 5, further comprising a preservative in an amount effective to preserve the stability of the composition.

7. A composition according to any one of claims 1 to 6, further comprising an organic acid in an amount effective to maintain a vaginal pH level from about 3.5 to about 5.5.

8. A composition according to any one of claims 1 to 7, further comprising at least one antioxidant.

9. A composition according to claim 8, wherein said antioxidant is selected from ascorbyl palmitate, benzoic acid, butylated hydroxytoluene, butylated hydroxyanisole, sodium bisulfite, sodium metabisulfite, maleic acid, propyl gallate or mixtures thereof.

10. A composition according to any one of claims 1 to 9, further comprising at least one chelator.

11. A composition according to claim 10 wherein said chelator is selected from the group consisting of ethylenediaminetetraacetic acid and salts thereof.

12. A composition according to any one of claims 1 to 11, further comprising an effective amount of a local anesthetic.

13. A composition according to any one of claims 1 to 12, further comprising a plant extract, wherein said plant extract is selected from aloe, witch hazel, chamomile, hydrogenated soy oil, oat extract, vitamin A, D and E, corticosteroids, sassafras oil and mixtures thereof.

14. A composition according to any one of claims 1 to 13, further comprising a pH adjustor.

15. Use of a composition according to any one of claims 1 to 14 for providing personal lubrication to the skin of an individual.
